(19) Europäisches Patentamt

European Patent Office

Office européen des brevets

(11) **EP 1 149 598 A2**

(12) ## EUROPEAN PATENT APPLICATION

(43) Date of publication:
**31.10.2001 Bulletin 2001/44**

(51) Int Cl.$^7$: **A61L 29/06**, A61L 29/04

(21) Application number: **01303819.5**

(22) Date of filing: **26.04.2001**

(84) Designated Contracting States:
**AT BE CH CY DE DK ES FI FR GB GR IE IT LI LU MC NL PT SE TR**
Designated Extension States:
**AL LT LV MK RO SI**

(30) Priority: **27.04.2000 JP 2000127604**

(71) Applicant: **TERUMO KABUSHIKI KAISHA Tokyo (JP)**

(72) Inventors:
• **Urakawa, Ryuichi**
  **Achigarakami-gun, Kanagawa (JP)**
• **Zushi, Yasunobu**
  **Achigarakami-gun, Kanagawa (JP)**

(74) Representative: **Harrison, David Christopher et al**
  **MEWBURN ELLIS**
  **York House**
  **23 Kingsway**
  **London WC2B 6HP (GB)**

(54) **Catheter and medical tube**

(57) A catheter is made of a melt-extruded polymeric material consisting of non-polyvinyl chloride. The catheter has a loss tangent not less than 0.15, when the dynamic viscoelasticity thereof is measured at 25°C in a longitudinal vibration thereof. The catheter has a kinking resistance not less than 25mm, when the catheter having a length of 70mm is used under a dry condition at room temperature.

A medical tube is made of a polymeric material consisting of non-polyvinyl chlorides. The medical tube consists of a compound of two or more kinds of polymeric materials. The medical tube has a loss tangent not less than 0.15, when the dynamic viscoelasticity thereof is measured at 25°C in a longitudinal vibration thereof. The medical tube has a kinking resistance not less than 25mm, when the medical tube having a length of 70mm is used under a dry condition at room temperature.

F I G. 2

**Description**

BACKGROUND OF THE INVENTION

The present invention relates to a catheter that is used by inserting it into a human body. The catheter is composed of a polymeric material consisting of non-polyvinyl chloride and has high kink resistance.

The present invention also relates to a medical tube that is composed of a polymeric material consisting of non-polyvinyl chloride and has high kink resistance.

The following disposable catheters are currently used: a urethral catheter, an enteric nutrition catheter, a bile duct catheter, a stomach catheter, a bronchia tube, a central venous catheter, a drainage tube and a suction catheter. As highfunction catheters, an angiography catheter, intracardiac catheter, a dilation catheter, a thrombus removal catheter, an epidural catheter and an endoscope catheter are currently used.

The disposable catheter is hitherto made of polyvinyl chloride that is inexpensive, excellent in its kinking resistance and flexible to some extent. However, it generates dioxin when it is burnt after use, which is at stake. Thus, there is a demand for the development of a catheter made of a material other than the polyvinyl chloride.

The property of each kind of the catheter is set in consideration of its purpose. For example, the urethral catheter is used to discharge urine directly to a toilet stool or to a urination bag by holding it in the urethra. Therefore, it is necessary to secure a lumen in a portion of the urethral catheter held in the human body and in a portion thereof exposed to the outside. The entire urethral catheter is also required to have kinking resistance so that it does not interfere with urination. In particular, a portion of the urethral catheter exposed to the outside and the boundary thereof between the human body and its exposed portion are required to have kinking resistance. Any kind of the catheter that is held in the human body is required to have the kinking resistance.

To improve the kinking resistance, a catheter that is made thinner by reducing its inner diameter is manufactured. Because the inner diameter of the catheter is small, it is incapable of keeping a high flow rate. Thus, it takes long time to feed discharged urine. To insert the catheter into the human body with high operability, it is desirable that the catheter is appropriately rigid. On the other hand, to prevent the tissues of the catheter-held portion of the human body from being damaged when the catheter is inserted thereinto, it is desirable that the catheter is appropriately flexible.

The medical tube includes a tube used to introduce a substance into the human body or discharging it therefrom and a catheter used for examination or treatment of a required portion of the human body by inserting it thereinto.

The medical tube includes catheters such as the urethral catheter, the stomach catheter and the suction catheter; tubes such as an infusion tube, an enteric nutrition catheter, a peritoneal dialysis tube, a transfusion tube and a tube connected to a urethral catheter to introduce urine into a urine storage bag; tubes used for a blood circuit for hemodialysis, a blood circuit for a pump-oxygenator and a blood circuit for blood plasma exchange; and tubes for transporting substances in a medical field. The tube used to transport substances in the medical field includes a tube mounted on a multiple blood bag and a tube for connecting an evacuator and a catheter to each other.

Most medical tubes are hitherto made of polyvinyl chloride that is inexpensive and excellent in its kinking resistance and flexible to some extent. However, it generates dioxin when it is burnt after use, which is at stake. Further in recent years, it is suspected that a plasticizer contained in soft polyvinyl chloride has an extrinsic incretion-disturbing action.

To solve the above-described problem, it is an object of the present invention to provide a catheter that is composed of a polymeric material consisting of non-polyvinyl chloride and have high kinking resistance, appropriate flexibility and appropriate rigidity.

It is another object of the present invention to provide a medical tube that is composed of a polymeric material consisting of non-polyvinyl chloride and have high kinking resistance, appropriate flexibility and appropriate rigidity.

SUMMARY OF THE INVENTION

The object of this invention is to provide a catheter which is made of a melt-extruded polymeric material consisting of non-polyvinyl chloride having a loss tangent not less than 0.15 when a dynamic viscoelasticity thereof is measured at 25°C in a longitudinal vibration thereof and having a kinking resistance not less than 25mm when said catheter having a length of 70mm is used under a dry condition at room temperature.

Further, the object of this invention is to provide a medical tube which is made of a compound of two or more kinds of polymeric materials each consisting of non-polyvinyl chloride having a loss tangent not less than 0.15 when a dynamic viscoelasticity thereof is measured at 25°C in a longitudinal vibration thereof and having a kinking resistance not less than 25mm when said medical tube having a length of 70mm is used under a dry condition at room temperature.

BRIEF DESCRIPTION OF THE DRAWINGS

Fig. 1 is an explanatory view showing a compression tester that is used to

measure a kinking resistance and a maximum push-fit strength.

Fig. 2 is an explanatory view showing the compression tester that is used to measure the kinking resistance and the maximum push-fit strength.

Fig. 3 is an explanatory view for explaining results of the kinking resistance and the maximum push-fit strength.

Fig. 4 shows the catheter, of an embodiment of the present invention, which is used as a urethral catheter.

Fig. 5 is an enlarged sectional view showing a front part of the catheter shown in Fig. 4.

Fig. 6 shows the catheter, of another embodiment of the present invention, which is used as a urethral catheter.

DETAILED DESCRIPTION OF THE PREFERRED EMBODIMENTS

An embodiment of the catheter of the present invention will be described below.

A catheter of the present invention is made of a melt-extruded polymeric material consisting of non-polyvinyl chloride. In other words, the catheter of the present invention is made of a non-polyvinyl chloride melt-extruded polymeric material. The loss tangent of the catheter is not less than 0.15, when its dynamic viscoelasticity is measured at 25°C in its longitudinal vibration. The kinking resistance of the catheter having a length of 70mm is not less than 25mm, when the catheter is used under a dry condition at room temperature. The dynamic viscoelasticity of the catheter in its longitudinal vibration is measured by using a strip formed by longitudinally cutting the catheter to a predetermined length.

By using the catheter having the above values as the material for the catheter, it is possible to sufficiently prevent the catheter of the present invention from being kinked by an external force applied thereto, when it is inserted into a human body at an operating temperature range.

The reason the value of the loss tangent is set to the above range is as follows: if the loss tangent at 25°C is not less than 0.15, an external force applied to the catheter is relieved and thus the catheter is prevented from kinking. On the other hand, if the catheter has the loss tangent less than 0.15 at 25°C, the catheter yields to the external force applied thereto and is liable to kink. Thereby there is a possibility that the catheter interferes with an operation of inserting the catheter into the human body and that its lumen is dosed. Further, if the catheter has the loss tangent less than 0.15 at 25°C, a stress applied to the front portion of the catheter in its lateral direction is not relieved (energy loss does not occur). Consequently, there is a possibility that the front portion of the catheter leaps and interferes with the catheter insertion operation. Further, if the catheter having the loss tangent less than 0.15 is used as a urethral catheter, there is a possibility that the front portion of the urethral catheter leaps and that urine which has attached to the front portion thereof is scattered in the periphery. Thus, such a urethral catheter is unsanitary.

Polyvinyl chloride that is generally used for a medical tube has a loss tangent more than 0.15. Therefore, the catheter made of the polyvinyl chloride is not repulsive for an external force applied thereto when it is inserted into the human body, thus giving a user a soft feeling. If the catheter has a loss tangent less than 0.15 at 25°C, the external force applied to the catheter is not converted enough into energy that is consumed as a micro-Brownian motion or the like but stored therein. Consequently, the catheter will return to the original shape rapidly in correspondence to a quantity of energy applied thereto. As a result, the catheter will leap at its front end, thus not giving the user a flexible feeling. On the other hand, if the catheter has a loss tangent more than 0.15, the external force applied to the catheter is appropriately converted into the energy or the like consumed by a micro-Browning motion of a polymeric material forming the catheter. Thus, the catheter does not rapidly return to its original shape and does not leap at its front portion. Consequently, the catheter is as flexible as the catheter made of the polyvinyl chloride.

It is also preferable that the loss tangent of the material for the catheter is less than 0.5. In the case where the catheter made of the non-polyvinyl chloride has a loss tangent less than 0.5, the catheter is not too flexible. That is, the catheter is not limp. Therefore, the loss tangent of the material for the catheter is favorably in the range of 0.15 to 0.5. It is also preferable that the loss tangent of the material for the catheter is less than 0.4. Therefore, the loss tangent of the material for the catheter is favorably in the range of 0.15 to 0.4, furthermore favorably in the range of 0.15 to 0.35.

The material for the catheter of the present invention has a high kinking resistance. Thus, the catheter has a kinking resistance in a certain extent in the case where its inner diameter is made larger, i.e., in the case where the catheter is thinned. Favorably, the wall thickness of the catheter is in the range of 0.4 to 0.9mm when the catheter size is 12F. In the case where the wall thickness of the catheter is not less than 0.4mm, it has a high degree of kinking resistance. In the case where the wall thickness of the catheter is not more than 0.9mm, the lumen of the catheter is capable of having a sufficient diameter. More favorably, the wall thickness of the catheter is in the range of 0.6 to 0.8mm. Favorably, the ratio of the outer diameter of the catheter to its wall thickness is in the range of 1:0.1 to 1:0.24. In the case where the ratio is more than 1:0.1, the catheter has a high kinking resistance. In the case where the ratio is less than 1:0.24, the lumen of the catheter is capable of having a sufficient diameter. More favorably, the ratio is in the range of 1:0.15 to 1:0.2.

In the case where the kinking resistance (when the length of the catheter is 70mm) of the catheter is not less than 25mm under a dry condition at room temperature (region of operating temperature), the catheter is capable of having a large-diameter lumen securely when the catheter is inserted into the human body and when it is inside and outside the human body. If the catheter has a kinking resistance less than 25mm, the catheter does not have a high degree of kinking resistance. In this case, there is a possibility that the lumen of the catheter is dosed and that a kinked portion of the catheter presses and damages the catheter-contact portion of the human body.

It is preferable that a maximum push-fit strength (when the length of the catheter is 70mm) of the catheter of the present invention is in the range of 0.5N to 5N under a dry condition at room temperature. In the case where the maximum push-fit strength is in this range, the catheter is appropriately rigid to give it high operability in inserting it into the human body. The maximum push-fit strength means a maximum load applied to the catheter when the catheter starts to flex after the catheter is compressed longitudinally in a compression test described later. Thus, it can be said that the maximum push-fit strength indicates the yielding resistance of the catheter to an external force applied thereto in a catheter insertion operation.

In the case where the maximum push-fit strength is not less than 0.5N under a dry condition at room temperature, the catheter does not yield easily to the external force applied thereto and has high operability. In the case where the maximum push-fit strength is not more than 5N, the catheter is not excessively rigid. Thus, there is no possibility that the catheter gives a patient a feeling of physical disorder or damages the catheter-contact portion of the human body. The maximum push-fit strength is favorably in the range of 1 to 3N and more favorably in the range of 1 to 2N under a dry condition at room temperature. The catheter having the maximum push-fit strength in this range can be easily inserted into the human body.

It is favorable that the Shore hardness of the polymeric material for the catheter is in the range of 70 to 95A. The catheter having the Shore hardness not more than 70A is too flexible and may interfere with an operation of inserting it into the human body. The catheter having the Shore hardness more than 95A can be easily inserted into the human body, but is so hard that there is a possibility that the catheter gives the patient pain or physical disorder and damages the medical tube-contact portion of the human body. It is more favorable that the Shore hardness of the polymeric material for the catheter is in the range of 80 to 90A.

The catheter of the present invention is particularly effective by using it as the urethral catheter, but may be used as the following catheters and tubes: an enteric nutrition catheter, a bile duct catheter, a stomach catheter, a bronchia tube, a central venous catheter, a drainage tube, a suction catheter, an angiography catheter, intracardiac catheter, a dilation catheter, a thrombus removal catheter, an epidural catheter and an endoscope catheter. Furthermore, melt-extruded polymeric materials consisting of non-polyvinyl chloride that is used to the catheter of the present invention can use it for a medical tube such as an infusion tube, a enteric nutrition catheter, a peritoneal dialysis tube, a transfusion tube and a tube connected to a urethral catheter to introduce urine into a urine storage bag; tubes used for a blood circuit for hemodialysis, a blood circuit for a pump-oxygenator and a blood circuit for blood plasma exchange; and tubes for transporting substances in a medical field. The tube used to transport substances in the medical field includes a tube mounted on a multiple blood bag and a tube for connecting an evacuator and a catheter to each other.

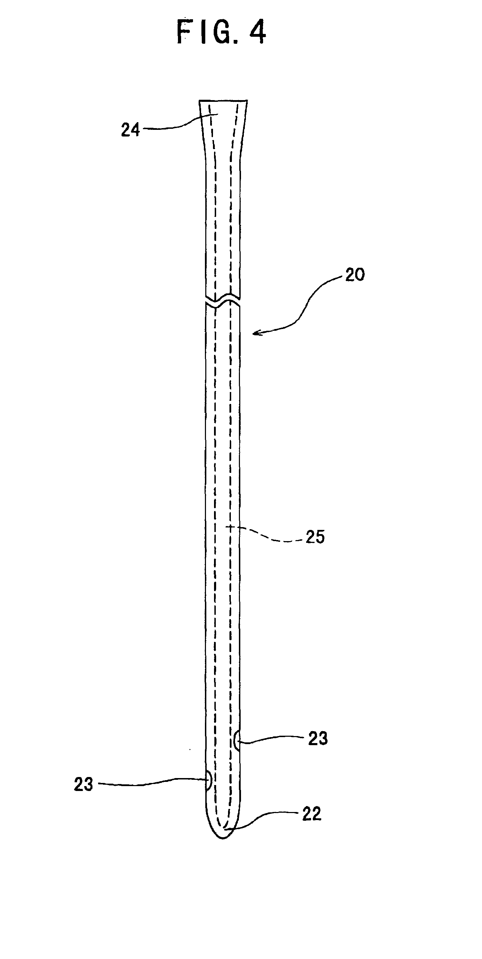

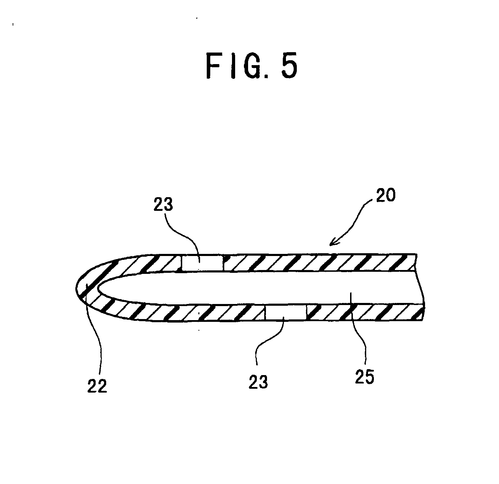

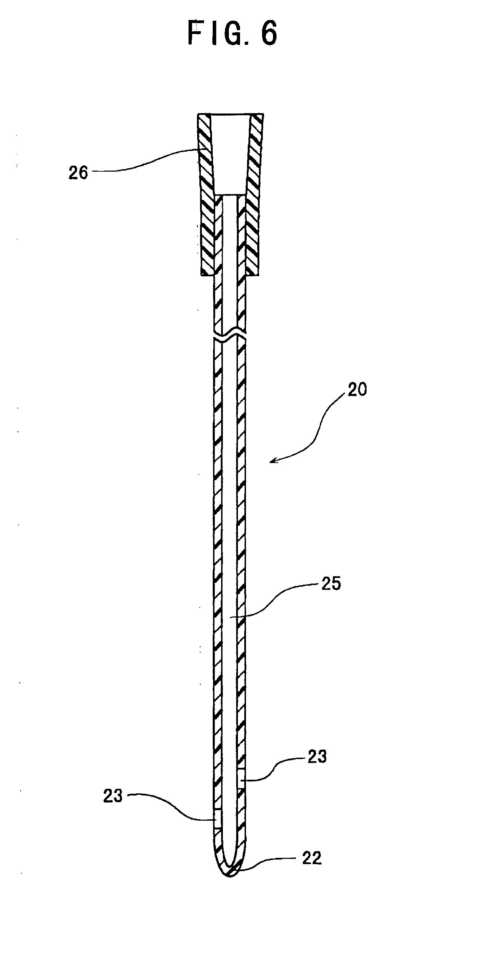

It is preferable that the urethral catheter has a conventional mode as shown in Fig. 4. As shown in Fig. 4 and 5 which is an enlarged sectional view of the front portion of a urethral catheter 20, the urethral catheter 20 has a closed front end 22, a side opening 23 formed at the front portion thereof, a rear portion 24 whose diameter gradually increases to the rear end of the urethral catheter 20 and which has an opening formed at the rear end of the urethral catheter 20 and a lumen 25 which extends from the opening formed at the rear end of the urethral catheter 20 to the front end thereof and is open at the side opening 23. Except the front portion and rear portion of the urethral catheter 20, the outer diameter of the urethral catheter 20 is almost uniform. As shown in Fig. 6, at the rear end of the urethral catheter 20, there may be provided an adapter 26 whose diameter increases gradually to its rear end.

The catheter of the present invention is formed of a polymeric material consisting of non-polyvinyl chloride. Thus, the catheter does not generate dioxin when it is burnt after use.

As the polymeric material consisting of the non-polyvinyl chloride, it is possible to use thermoplastic elastomers such as a polyurethane elastomer, a polyester elastomer, a polyamide elastomer, a polyolefin elastomer and a styrene elastomer. It is also possible to use a polybutadiene elastomer as the polymeric material consisting of the non-polyvinyl chloride. It is also possible to use a compound of two or more elastomers selected from among the polyurethane elastomer, the polyester elastomer, the polyamide elastomer, the polyolefin elastomer, the styrene elastomer and the polybutadiene elastomer as the melt-extruding material for the catheter of the present invention.

It is possible to use the polyurethane elastomer containing polyether (for example, polytetramethylene oxide), polyester (adipate or polycaprolactone) or polycarbonate as a soft segment and a component formed from diisocyanates such as tuluene diisocyanate, diphenylmethane diisocyanate, hexamethylene diisocyanate or isophorone diisocyanate as a hard segment.

It is preferable to compose the polyurethane elastomer consisting of both the diisocyanate and a chain extender as a hard segment; and polyglycol as a soft segment. The polyester-segmented polyurethane elastomer is a kind of the polyurethane elastomer of polyether type.

As the diisocyanate component, it is possible to use aromatic diisocyanate such as 4,4'-diphenylmethane diisocyanate (MDI), 3,3'-diphenylmethane diisocyanate, toluene diisocyanate and the like; alicyclic diisocyanate such as 4,4'-dicyclohexylmethane diisocyanate (HMDI), isophorone diisocyanate and the like; and aliphatic diisocyanate such as 1,6-hexamethylene diisocyanate and the like. The MDI and the HMDI are most favorable of the above diisocyanate components.

As the chain extender, it is possible to use low-molecular-weight diol such as 1,4-butanediol, ethylene glycol, diethylene glycol, triethylene glycol, propylene glycol and 1,6-hexanediol. The 1,4-butanediol is most favorable of these chain extenders. It is possible to use ethylenediamine, butylenediamine and hexamethylenediamine as the chain extender. In this case, urea bond may be partially introduced into the polyurethane elastomers.

As the polyglycol component, it is possible to use polyether glycol, polycarbonate glycol, polycaprolactone glycol and polyadipate glycol. As the polyether glycol, it is possible to use polytetramethylene glycol, polyethylene glycol and polypropylene glycol; a copolymer thereof; or a mixture thereof.

It is favorable that the Shore hardness of the polyurethane for the catheter is in the range of 70 to 95A. The catheter having the Shore hardness less than 70A is too flexible and may interfere with an operation of inserting it into the human body. The catheter having the Shore hardness more than 95A can be easily inserted into the human body but is too hard, thus having a possibility of giving the patient pain or physical disorder and damaging the medical tube-contact portion of the human body. It is more favorable that the Shore hardness of the polymeric material for the catheter is in the range of 80 to 90A.

To improve resistance of a polyurethane material to hydrolysis and oxidation-caused deterioration, the polyurethane material may contain a hydrolysis inhibitor and an antioxidant.

To provide the catheter having a high kinking resistance, it is preferable that the polyurethane forming the catheter is comprised of a hard segment consisting of MDI and 1,4-butanediol and a soft segment consisting of polycarbonate glycol. It is also preferable that the polyurethane has a Shore hardness in the range of 70 to 95A.

It is preferable that the polyurethane forming the catheter is comprised of a hard segment consisting of HMDI and 1,4-butanediol and a soft segment consisting of polytetramethylene glycol. It is also preferable that the polyurethane has a Shore hardness in the range of 70 to 95A.

It is preferable that the polyurethane forming the catheter is comprised of a hard segment consisting of MDI and 1,4-butanediol and a soft segment consisting of polytetramethylene glycol or a mixture of polytetramethylene glycol, polypropylene glycol and polyethylene glycol or a copolymer thereof. It is also preferable that the polyurethane has a Shore hardness in the range of 70 to 95A. The catheter having the above-described construction is excellent in its kinking resistance and can be inserted into the human body easily and reliably.

The polyolefin elastomer comprises the polyolefin as a hard segment and an olefin rubber as a soft segment. As the polyolefin, it is possible to use a unipolymer of $\alpha$-olefin such as ethylene, propylene, 1-butene and 4-methyl-1-pentene or a random copolymer thereof or a block copolymer thereof. As the olefin rubber, it is possible to use a copolymer rubber consisting of ethylene and $\alpha$-olefin, an ethylene-$\alpha$-olefin-unconjugated diene copolymer rubber and the like. As the polyolefin elastomer, it is possible to use a tri-block type thermoplastic elastomer such as Dynalon 6200P (produced by JSR Corp.), Thermolan (produced by Mitsubishi chemical Corp.), Milastomer (produced by Mitsui petrochemical industries Co.,ltd.) and Sumitomo TPE (produced by Sumitomo chemical Co.,ltd.) each having crystalline polyolefin at both ends of the main chain of an ethylene-butadiene copolymer.

As the olefin elastomer, it is possible to use an elastomer having crystalline polyolefin as a hard segment and uncrystalline polyolefin as a soft segment. It is preferable that the crystalline polyolefin is formed of polyethylene. It is particularly preferable that the uncrystalline polyolefin is formed of a copolymer of ethylene and other monomer.

As examples of the polyolefin elastomer, the following copolymers can be used: an ethylene-vinyl acetate copolymer; an ethylene-acrylic ester copolymer; an ethylene-$\alpha$-olefin copolymer formed by copolymerizing ethylene with $\alpha$-olefin such as propylene, 1-butene, 1-hexene, 1-octen or the like having not less than three carbon atoms; an ethylene-conjugated diene copolymer formed by copolymerizing ethylene with a conjugated diene monomer such as butadiene, isoprene or the like; ethylene-cydic polyolefin copolymer formed by copolymerizing ethylene with cyclic olefin such as cyclopentene, cyclopentadiene, cydohexene or the like; an ethylene-aromatic vinyl copolymer formed by copolymerizing ethylene with a aromatic vinyl monomer such as styrene, p-methylstyrene, m-methylstyrene, o-methylstyrene, o-t-butylstyrene, m-t-butylstyrene, p-t-butylstyrene, p-chlorostyrene, $\alpha$-methylstyrene or the like.

It is preferable that the amount of ethylene of the ethylene copolymer is in the range of 50 mol % to 95 mol %. When the amount of ethylene is not less than 50 mol %, it is possible for the ethylene part to crystallize. Such materials provide a medical tube with a sufficient strength and rubber elasticity by the melt-extrusion method. On the other hand, when the amount of the ethylene is not more than 95 mol %, the ethylene part can crystallize strongly and such materials show insufficient flexibility.

As the polyester elastomer, it is possible to use a polyethylene terephthalate-poly (tetramethylene oxide) glycol block copolymer, a polyethylene terephthalate/isophthalate-poly (tetramethylene oxide) glycol block copolymer, a polybutylene terephthalate-poly(tetramethylene oxide) glycol block copolymer, a polybutylene terephthalate/ isophthalate-poly (tetramethylene oxide) glycol block copolymer, a polybutylene terephthalate/decane dicarboxylate-poly(tetramethylene oxide) glycol block copolymer, a polybutylene terephthalate-poly(propylene oxide/ethylene oxide) glycol block copolymer, a polybutylene terephthalate/isophthalate-poly (propylene oxide/ethylene oxide) glycol block copolymer, a polybutylene terephthalate/decanedicarboxylate-poly(propylene oxide/ethylene oxide) glycol block copolymer, a polybutylene terephthalate-poly (ethylene oxide) glycol block copolymer, a polybutylene terephthalate-polyethylene adipate block copolymer, a polybutylene terephthalate-polybutylene adipate block copolymer, a polybutylene terephthalate-polybutylene sebacate block copolymer and a polybutylene terephthalate-poly-ε-caprolactone block copolymer.

The polyamide elastomer comprised of polyamide as a hard segment and polyether or polyester as a soft segment. As the polyamide, it is preferable to use nylon 6, 66,11,12, or 610 or a mixture containing one or more thereof as its main component. As the polyether, polytetramethylene oxide is preferable.

As the styrene elastomer, it is possible to use a styrene-butadienebutylene-styrene copolymer, a styrene-(ethylene-butylene)-styrene copolymer (SEBS), a styrene-butadiene-styrene copolymer (SBS), a styrene-ethylenepropylene-styrene copolymer (SEPS) and the like. As the SEBS, Clayton G1657 (produced by Shell chemical Corp.) can be used.

As the styrene elastomer, it is possible to use a copolymer comprising styrene as a hard segment and an unsaturated olefin monomer such as isobutene, pentene and 4-methylpentene, conjugated diene such as butadiene and isoprene and hydrogenated conjugated diene as a soft segment.

A random copolymer or a block copolymer can be used as the copolymer. As the block copolymer consisting of the styrene elastomer, it is possible to use the following block copolymers having 1,4-conjugated diene: a styrene-butadiene-styrene copolymer (SBS), a styrene-isoprene-styrene copolymer (SIS) and a styrene-(butadiene-isoprene)-styrene copolymer (SBIS). It is also possible to use the following block copolymers formed by hydrogenating the conjugated diene of each of the above block copolymers: a styrene-(ethylene-butylene)-styrene copolymer (SEBS), a styrene-(ethylene-propylene)-styrene copolymer (SEPS) and a styrene-(ethylene-ethylene-propylene)-styrene copolymer (SEEPS). These block copolymers are commercially available as SIS (produced by JSR Corp.), Septon (produced by Kuraray Co.,ltd.) and Clayton (produced by Shell chemical Corp.).

As the block copolymer consisting of the styrene elastomer, it is preferable to use a block copolymer formed by hydrogenating the isoprene block of the styrene-isoprene-styrene block copolymer. It is preferable that the styrene-isoprene-styrene tri-block copolymer consisting of the polyisoprene block which has 3,4-isoprene repeating unit at not less than 45% and more favorable at not less than 60%.The block copolymer is commercially available under the trade name of Hybrar (produced by Kuraray Corp.).

As the random copolymer consisting of the styrene elastomer, it is possible to use a random copolymer formed by hydrogenating a styrenebutadiene random copolymer. The random copolymer is commercially available as Dinalon (produced by JSR Corp.).

The styrene elastomer includes a compound formed by adding polyolefin and process oil or the like to the styrene elastomer such as SEBS or the like. As the styrene elastomer containing the SEES and the SEPS, Rubberon (produced by Mitsubishi Chemicals Corp.), Septon compound (produced by Kuraray Corp.), Leostomer (produced by Riken Vinyl Industry Co.,ltd.) and Actymer (produced by Riken Vinyl Industry Co.,ltd.) or the like are commercially available.

The polybutadiene elastomer contains crystalline polybutadiene as a hard segment and uncrystalline polybutadiene as a soft segment. It is possible to use syndiotactic 1,2 polybutadiene as the polybutadiene elastomer commercially available as RB810 (produced by JSR Corp.).

As the melt-extruded material for the catheter of the present invention, it is also possible to use a mixture of two or more elastomers optionally selected from among the polyurethane elastomer, the polyester elastomer, the polyamide elastomer, the polyolefin elastomer, the styrene elastomer and the polybutadiene elastomer.

In this case, supposing that the loss tangent of one selected polymeric material at 25°C is less than 0.15, it is preferable to add one or more elastomers to the polymeric material to make the loss tangent of a mixture of two or more polymeric materials not less than 0.15 at 25°C. Thereby it is possible to obtain a catheter having a high kinking resistance. In this case, it is preferable that the loss tangent of at least one of the added polymeric materials is not less than 0.3 when its dynamic viscoelasticity is measured at 25°C.

As a styrene elastomer having a loss tangent not less than 0.3 at 25°C, it is preferable to use a styrene-isoprene-styrene tri-block copolymer containing the 3,4 vinyl bond of the isoprene block at not less than 60%. It is also preferable to use a hydrogenated tri-block copolymer formed by hydrogenating the isoprene block of the styrene-isoprene-styrene tri-block copolymer. The styrene-isoprene-styrene tri-block copolymer is commercially available as Hybrar 5127 (produced by Kuraray Corp.)

As another elastomer having a loss tangent not less than 0.3 at 25°C, it is preferable to use an olefin elastomer consisting of ethylene and other monomer.

The olefin elastomer is commercially available as Index (produced by Dow Chemical Corp.). As still another elastomer having a loss tangent not less than 0.3 at 25°C, it is preferable to use a polyurethane elastomer consisting of MDI and 1,4-butanediol as a hard segment and polycarbonate glycol as a soft segment and having a Shore hardness of 90A; and a polyurethane copolymer consisting of HMDI and 1,4-butanediol as a hard segment and polytetramethylene glycol as a soft segment and having a Shore hardness of 90A.

It is necessary that two or more kinds of to-be-mixed polymeric materials are compatible with each other to allow a catheter formed of a compound thereof to have a demanded kinking resistance, strength, transparency and flexibility as a medical tube.

The use of compatible polymeric materials prevents macro-dispersion of domains in resin. As the stress does not concentrate on an interface between the domains, the tube can have a high kinking resistance and a high tensile strength. Further, because light is hardly scattered on the macro-interface between the domains, the catheter is highly transparent.

In each of the polyurethane elastomer, the polyester elastomer, the polyamide elastomer, the polyolefin elastomer, the styrene elastomer and the polybutadiene elastomer, elastomers of the same kind are compatible with each other. Even in elastomers of different kinds, the styrene elastomer and the olefin elastomer; the styrene elastomer and the polybutadiene elastomer; the polyurethane elastomer, the polyester elastomer and the polyamide elastomer are compatible with one other, respectively.

The loss tangent of the catheter is obtained by the following method of measuring its dynamic viscoelasticity in its longitudinal vibration:

A method of measuring the dynamic viscoelasticity of the polymeric material by means of torsional vibration is known. But this method is incapable of evaluating the kinking resistance of the melt-extruded catheter. It is preferable to measure its dynamic viscoelasticity in its longitudinal vibration, that is, stretching and compressing the catheter longitudinally in order to evaluate the kinking resistance of the catheter. When an external force is applied to the catheter, the catheter yields thereto, i.e., the catheter kinks. When the catheter kinks, its outer side stretches, whereas its inner side compresses.

The dynamic viscoelasticity of the polymeric material in its longitudinal vibration was measured on a dynamic viscoelastic analyzer DVA-225 manufactured by IKT Co. Ltd. by temperature rise method in the air at a measuring frequency of 10 Hz at a temperature rise rate of 5°C/min. A specimen was formed by cutting the catheter to a strip having 2mm in its widthwise direction and 25mm in its longitudinal direction.

Based on measured dynamic storage elastic modulus (E') and dynamic loss elastic modulus (E"), a loss tangent(tanδ) was estimated by the following equation:

$$E^* = E' + iE''$$

$$\tan\delta = E''/E'$$

where E* is a complex modulus.

The kinking resistance and the maximum push-fit strength were measured by the following method:

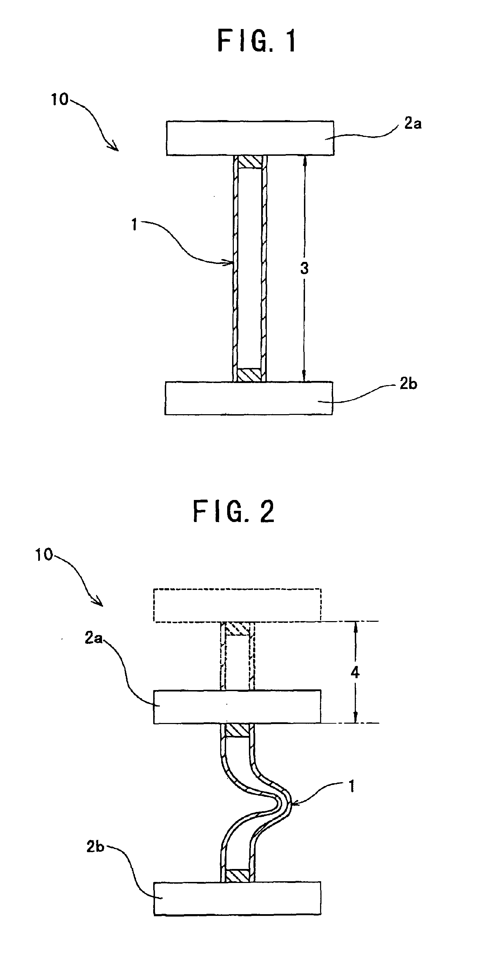

The kinking resistance and the maximum push-fit strength were measured by using a compression tester 10 shown in Figs. 1 and 2. The compression tester 10 has a clamp 2a so installed at an upper portion thereof as to be movable vertically at a constant speed and a clamp 2b fixed to a lower portion thereof. A catheter 1 having a predetermined length was set between the clamps 2a and 2b. The compression tester 10 was so constructed that by compressing the catheter 1 axially, the change of a load applied to the catheter 1 was recorded on a chart. The kinking resistance and push-fit strength of the catheter 1 having a length 3 of 70mm were measured at a compression speed of 100mm/min. at a room temperature (25°C, dry) as follows. The catheter 1 used in this test had a size 12F having an inner diameter of φ2.6mm and an outer diameter of φ4.0mm.

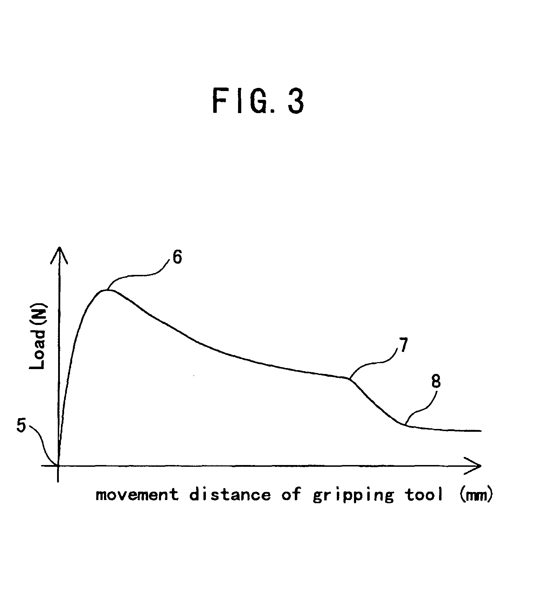

As the catheter 1 was compressed axially, as shown in Figs. 1 and 2, a load applied to the catheter 1 changes. The change of the load is shown on the chart of Fig. 3. Upon axial compression of the catheter 1, the load applied to the catheter 1 increases instantaneously. When the catheter 1 starts to bend, the load decreases (flexure start point 6). The load measured when the catheter 1 has started to bend is the maximum push-fit strength. As the compression continues, the lumen of the catheter 1 is crushed and starts to be dosed (kinked). Thereby there is a great change in the reduction degree of the load to record an inflection point (kink start point 7) on the chart. Simultaneously with the closing of the lumen of the catheter 1, the load is applied thereto constantly. At this time, an inflection point (kink point 8) is recorded on the chart. In the compression test, an operator measures the movement distance (mm) 4 of the clamp 2a from a position corresponding to a start point (start point 5) to a position corresponding to the closing (kink point 8) time of the lumen of the catheter 1 to determine the kinking resistance of the catheter 1. In this manner, the kinking resistance and the maximum push-fit strength are measured simultaneously.

The examples of the medical tube of the present invention will be described below.

The medical tube of the present invention is formed by melt-extruding a polymeric material consisting of non-polyvinyl chloride. More specifically, the medical tube is made of a mixture of two or more kinds of polymeric materials. The loss tangent of the medical tube is not less than 0.15 when its dynamic viscoelasticity is measured at 25°C in its longitudinal vibration.

The kinking resistance of the medical tube having a length of 70mm is not less than 25mm under a dry condition at room temperature.

By using the medical tube having the above values as the material for the tube, it is possible to sufficiently prevent the medical tube of the present invention from being kinked by an external force applied thereto, when it is inserted into the human body at an operating temperature.

The reason for that the loss tangent is set to not less than 0.15 is as follows: in the case where the loss tangent at 25°C is not less than 0.15, an external force applied to the medical tube is relieved and thus the medical tube is prevented from kinking. On the other hand, if the loss tangent at 25°C is less than 0.15, the medical tube yields to the external force and is liable to kink. Thereby there is a possibility that the medical tube interferes with an operation of inserting the medical tube into the human body and its lumen is closed. Further, if the loss tangent at 25°C is less than 0.15, a stress applied to the front portion of the medical tube in its lateral direction is not relieved (energy loss does not occur). Consequently, there is a possibility that the front portion of the medical tube leaps and interferes with a medical tube insertion operation. Further, if the medical tube having the loss tangent less than 0.15 is used as a urethral catheter, there is a possibility that the front portion of the medical tube leaps and that urine which has attached to the front portion of the medical tube is scattered in the periphery. Thus, such a catheter is unsanitary.

It is preferable that the loss tangent of the material for the medical tube is less than 0.5. In the case where the loss tangent of the medical tube is less than 0.5, the medical tube is not too flexible. That is, the catheter is not limp. Therefore, the loss tangent of the material for the medical tube is favorably in the range of 0.15 to 0.5, more favorably in the range of 0.15 to 0.4, furthermore favorably in the range of 0.15 to 0.35.

The material for the medical tube of the present invention has a high kinking resistance. Thus, the medical tube can display the kinking resistance in a certain extent in the case where its inner diameter is made larger, i.e., when it is thinned. Favorably, the wall thickness of the medical tube is in the range of 0.4 to 0.9mm. In the case where the wall thickness of the medical tube is not less than 0.4mm, it has a high degree of kinking resistance. In the case where the wall thickness of the medical tube is not more than 0.9mm, the lumen of the medical tube is capable of having a sufficient diameter. More favorably, the wall thickness of the medical tube is in the range of 0.6 to 0.8mm. Favorably, the ratio of the outer diameter of the medical tube to the wall thickness is in the range of 1:0.1 to 1:0.24. In the case where the ratio is more than 1:0.1, the catheter has a high degree of kinking resistance. In the case where the ratio is less than 1:0.24, the lumen of the catheter is capable of having a sufficient diameter. More favorably, the ratio is in the range of 1:0.15 to 1:0.2.

It is preferable that the kinking resistance (when the length of the medical tube is 70mm) of the medical tube is not less than 25mm under a dry condition at room temperature (region of operating temperature). In this case, the medical tube is capable of securing a large-diameter lumen when the tube is operated and used. In the case where the kinking resistance of the medical tube is not less than 25mm, the medical tube has a high degree of kinking resistance.

It is preferable that the maximum push-fit strength (when the length of the medical tube is 70mm) of the medical tube of the present invention is in the range of 0.5N to 5N under a dry condition at room temperature. In the case where the maximum push-fit strength is in this range, the medical tube is appropriately rigid, the medical tube has high operability in inserting the medical tube into the human body. The maximum push-fit strength means a load applied to the medical tube when the medical tube starts to leap after it is inserted into the human body longitudinally. It can be said that the maximum push-fit strength indicates the yielding resistance of the medical tube to an external force applied thereto in a medical tube insertion operation.

In the case where the maximum push-fit strength is not less than 0.5N under a dry condition at room temperature, the medical tube does not yield easily to the external force applied thereto and has high operability. If the maximum push-fit strength is less than 5N, the medical tube is not excessively rigid. Thus, there is no possibility that the medical tube gives a patient a feeling of physical disorder or damages the medical tube contact portion of the human body. The maximum push-fit strength is favorably in the range of 1 to 3N and more favorably in the range of 1 to 2N under a dry condition at room temperature. The medical tube having the maximum push-fit strength in this range can be easily inserted into the human body.

It is favorable that the Shore hardness of the polymeric material for the medical tube is in the range of 60 to 95A. The medical tube having the Shore hardness less than 60A is too flexible and may interfere with an operation of inserting it into the human body. The medical tube having the Shore hardness more than 95A can be easily inserted into the human body but is too hard. Thus, there is a possibility that the medical tube gives the patient pain or physical disorder and damages the medical tube-contact portion of the human body. It is more favorable that the Shore hardness of the polymeric material for the medical tube is in the range of 70 to 90A. It is most favorable that the Shore hardness thereof is in the range of 80 to 90A.

The medical tube of the present invention includes catheters; tubes such as an infusion tube, a enteric nutrition catheter, a peritoneal dialysis tube, a transfusion tube and a tube connected to a urethral catheter to introduce urine into a urine storage bag; tubes used for a blood circuit for hemodialysis, a blood circuit for a pump-oxygenator and a blood circuit for blood plasma exchange; and tubes for transporting substances in a medical field. The tube used to transport substances in the medical field includes a tube mounted on a multiple blood bag and a tube for connecting an evacuator and a catheter to each other.

The catheter of the present invention is particularly effective by using it as the urethral catheter, but may be used as the following catheters and tubes: a enteric nutrition catheter, a bile duct catheter, a stomach catheter, a bronchia tube, a central venous catheter, a drainage tube, a suction catheter, an angiography catheter, intracardiac catheter, a dilation catheter, a thrombus removal catheter, an epidural catheter and an endoscope catheter.

It is preferable that the urethral catheter has a conventional mode as shown in Fig. 4. As shown in Fig. 4 and Fig. 5 which is an enlarged sectional view of the front portion of a urethral catheter 20, the urethral catheter 20 has a closed front end 22, a side opening 23 formed at the front portion thereof, a rear portion 24 whose diameter gradually increases to the rear end of the urethral catheter 20 and which has an opening formed at the rear end of the urethral catheter 20 and a lumen 25 which extends from the opening formed at the rear end of the urethral catheter 20 to the front end thereof and is open at the side opening 23. Except the front and rear portions of the urethral catheter 20, the outer diameter of the urethral catheter 20 is almost uniform. As shown in Fig. 6, at the rear end of the urethral catheter 20, there may be provided an adapter 26 whose diameter increases gradually to its rear end.

The catheter of the present invention is formed of a polymeric material composed of non-polyvinyl chloride. Thus, the catheter does not generate dioxin when it is burnt after use.

The medical tube of the present invention is made of a compound of two or more kinds of polymeric materials. The polymeric material which produces the medical tube of the present invention has a loss tangent not less than 0.15 when its dynamic viscoelasticity is measured at 25°C in its longitudinal vibration.

One of two or more kinds of polymeric materials for the medical tube has a loss tangent favorably not less than 0.3 and more favorably not less than 0.5 when its dynamic viscoelasticity is measured at 25°C in its longitudinal vibration. It is favorable that one of two or more kinds of polymeric materials for the medical tube has a loss tangent not less than 0.3 when its dynamic viscoelasticity is measured at 25°C in its longitudinal vibration and that other polymeric material has a loss tangent not more than 0.13 when its dynamic viscoelasticity is measured at 25°C in its longitudinal vibration. It is more favorable that one of two or more kinds of polymeric materials for the medical tube has a loss tangent not less than 0.35 when its dynamic viscoelasticity is measured at 25°C in its longitudinal vibration and that the other polymeric material has a loss tangent not more than 0.10 when its dynamic viscoelasticity is measured at 25°C in its longitudinal vibration.

It is preferable that one of two or more kinds of polymeric materials is composed any one of 1,2-polybutadiene, an etylene copolymer and a propylene copolymer.

It is preferable that one of two or more kinds of polymeric materials is composed of a block copolymer of a polymer block A consisting of an aromatic vinyl monomer as its main component and a polymer block B consisting of a conjugated diene monomer as its main component or a block copolymer of the polymer block A and the hydrogenated polymer block B.

As a combination of polymeric materials, it is preferable that a compound of two or more kinds of polymeric materials consists of any one of the 1,2-polybutadiene, the ethylene copolymer, the propylene copolymer and the block copolymer of the polymer block A consisting of the aromatic vinyl monomer as its main component and the polymer block B consisting of the conjugated diene monomer as its main component or the block copolymer consisting of the polymer block A and the hydrogenated polymer block B.

The following compounds of two or more kinds of polymeric materials are preferable:

(1) A compound of the ethylene copolymer and the block copolymer of the polymer block A consisting of the aromatic vinyl monomer as its main component and the polymer block B consisting of the conjugated diene monomer as its main component or the block copolymer of the polymer block A and the hydrogenated polymer block B.
(2) A compound of the ethylene copolymer and the block copolymer of the polymer block A consisting of the aromatic vinyl monomer as its main component and the polymer block B consisting of the conjugated diene monomer as its main component or the block copolymer of the polymer block A and the hydrogenated polymer block B.
(3) A compound of the 1,2-polybutadiene and the block copolymer of the polymer block A consisting of the aromatic vinyl monomer as its main component and the polymer block B consisting of the conjugated diene monomer as its main component or the block copolymer of the polymer block A and the hydrogenated polymer block B.
(4) A compound of an polyester-segmented-polyurethane elastomer and a polycarbonate-segmented polyurethane elastomer.
(5) A compound of an ethylene-octene copolymer and an ethylene-styrene copolymer.

It is possible to add process oil such as paraffin oil containing as its main component and a plastic additive such as a stabilizer and a lubricant to the polymeric material for the medical tube of the present invention.

As the compound of the ethylene copolymer, it is possible to list an ethylene-vinyl acetate copolymer; an ethylene-acrylic ester copolymer; an ethylene-α-olefin copolymer formed by copolymerizing ethylene with propylene and α-olefin such as 1-butene, 1-hexene, 1-octen or the like having not less than three carbon atoms; an ethylene-conjugated diene copolymer formed by copolymerizing ethylene with a conjugated diene monomer such as butadiene, isoprene or the like; ethylene-cyclic polyolefin copolymer formed by copolymerizing ethylene with cyclic olefin such as cyclopentene, cyclopentadiene, cyclohexene or the like; an ethylene-aromatic vinyl copolymer formed by copolymerizing ethylene with a aromatic vinyl monomer such as styrene, p-methylstyrene, m-methylstyrene, o-methylstyrene, o-t-butylstyrene, m-t-butylstyrene, p-t-butylstyrene, p-chlorostyrene, α-methylstyrene or the like. As the copolymer of the ethylene and a monomer, the ethylene-aromatic vinyl copolymer is favorable. The ethylene-styrene copolymer is most favorable of the ethylene-aromatic vinyl copolymers. As the ethylene copolymer, an ethylene-octene copolymer and an ethylene-vinyl acetate copolymer are also favorable.

It is preferable that the amount of the ethylene of the ethylene copolymer is in the range of 50 mol % to 95 mol %. When the amount of the ethylene is not less than 50 mol %, it is possible to crystallize the ethylene without inhibiting and provide a tube made of the melt-extruded ethylene copolymer with a sufficient degree of strength and rubber elasticity. On the other hand, when the amount of the ethylene is not less than 95 mol %, the ethylene part can crystallize strongly and such materials show insufficient flexibility.

As the block copolymer composed of the polymer block A consisting of the aromatic vinyl monomer as its main component and the polymer block B consisting of the conjugated diene monomer as its main component, it is preferable to use styrene as the monomer of block A and butadiene or isoprene as the the monomer of block B. It is also preferable to use the hydrogenated copolymer thereof.

It is preferable to use the following block copolymers having 1,4-conjugated diene segment: a styrene-butadiene-styrene copolymer (SBS), a styrene-isoprene-styrene copolymer (SIS) and a styrene-(butadiene-isoprene)-styrene copolymer (SBIS). It is also possible to use the following block copolymers formed by hydrogenating the above-mentioned block copolymers: a styrene-(ethylene-butylene)-styrene copolymer (SEBS), a styrene-(ethylenepropylene)-styrene copolymer (SEPS) and a styrene-(ethylene-ethylenepropylene)-styrene copolymer (SEEPS). These block copolymers are commercially available as SIS (produced by JSR Corp.), Septon (produced by Kuraray Corp.) and Clayton (produced by Shell Chemical Corp.).

It is preferable to use a tri-block copolymer formed by hydrogenating the isoprene block of the styrene-isoprene-styrene tri-block copolymer. It is favorable that the styrene-isoprene-styrene tri-block copolymer consisting of the polyisoprene block which has 3,4-isoprene repeating unit at not less than 60%.

It is preferable that the mixing weight ratio between the ethylene copolymer and the block copolymer composed of the polymer block A consisting of the aromatic vinyl monomer as its main component and the polymer block B consisting of the conjugated diene monomer as its main component or the block copolymer of the polymer block A and the hydrogenated polymer block B is in the range of 10:90 to 95:5.

It is preferable that the mixing weight ratio between the propylene copolymer and the block copolymer of the polymer block A consisting of the aromatic vinyl monomer as its main component and the polymer block B consisting of the conjugated diene monomer as its main component or the block copolymer composed of the polymer block A and the hydrogenated polymer block B is in the range of 10:90 to 95:5.

It is preferable that the mixing weight ratio between the 1,2-polybutadiene and the block copolymer of the polymer block A consisting of the aromatic vinyl monomer as its main component and the polymer block B consisting of the conjugated diene monomer as its main component or the block copolymer composed of the polymer block A and the hydrogenated polymer block B is in the range of 10:90 to 95:5.

As the compound of the polymeric materials for the medical tube of the present invention, the compound of the polyester-segmented polyurethane elastomer and the polycarbonate-segmented polyurethane elastomer is also preferable.

It is preferable to use the polyester-segmented polyurethane elastomer consisting of 4,4'-diphenylmethane diisocyanate (MDI) and 1,4-butanediol as a hard segment and polycaprolactone glycol or polyadipate glycol as a soft segment. The polyester-segmented-polyurethane elastomer is commercially available as Miractoran E185PNAT (Nippon Miractoran Corp.).

It is preferable to use the polycarbonate-segmented polyurethane elastomer consisting of the 4,4'-diphenylmethane diisocyanate (MDI) and the 1,4-butanediol as a hard segment and polycarbonate glycol as a soft segment. The polycarbonate-segmented polyurethane elastomer is commercially available as Miractoran E985PNAT (Nippon Miractoran Corp.).

The polycarbonate-segmented polyurethane elastomer has a comparatively high loss tangent, whereas the polyester-segmented polyurethane elastomer has a comparatively low loss tangent. It is preferable that the mixing weight ratio between the polycarbonate-segmented polyurethane elastomer and the polyester-segmented polyurethane elastomer is in the range of 100:0 to 30:70. The polycarbonate-segmented polyurethane elastomer (commercial name: Miractoran E985PNAT) has a loss tangent of 0.318. The polyester-segmented-polyurethane elastomer (commercial name: Miractoran E185PNAT) has a loss tangent of 0.126. It is possible to obtain a polymeric material having a loss tangent of 0.223 by mixing the polycarbonate-segmented polyurethane elastomer and the polyester-segmented-polyurethane elastomer with each other at a weight ratio of 50:50.

As the compound of the polymeric materials for the medical tube of the present invention, a compound of an ethylene-octene copolymer and an ethylene-styrene copolymer is preferable. Each of the ethylene-octene copolymer and the ethylene-styrene copolymer may be a block copolymer or a random copolymer. The ethylene-styrene copolymer has a comparatively high loss tangent, whereas the ethylene-octene copolymer has a comparatively low loss tangent. It is preferable that the mixing weight ratio between the ethylene-styrene copolymer and the ethylene-octene copolymer is in the range of 100:0 to 20:80. One (produced by Dow Chemical Corp.) of the ethylene-styrene copolymers has a loss tangent of 0.545, whereas the ethylene-octene copolymer (Engage 8100 produced by Dupont Dow Elastomer Corp.) has a loss tangent of 0.09. It is possible to obtain a polymeric material having a loss tangent of 0.335 by mixing these two copolymers with each other at a weight ratio of 50:50.

It is preferable that one selected polymeric material for the medical tube of the present invention has a loss tangent of not less than 0.3 when its dynamic viscoelasticity is measured at 25°C. In this case, even when a polymeric material having a loss tangent of less than 0.15 at 25°C is mixed with the selected polymeric material, it is possible to make the loss tangent of the compound for the medical tube not less than 0.15 at 25°C.

As polymeric materials having a loss tangent not less than 0.3 at 25°C, it is possible to use the above-described ethylene copolymer. That is, the styrene-isoprene-styrene tri-block copolymer consisting of the polyisoprene which has 3,4-isoprene repeating unit at not less than 45%; the hydrogenated styrene-isoprene-styrene tri-block copolymer formed by hydrogenating the polyisoprene block of the styrene-isoprene-styrene tri-block copolymer; the ethylene-styrene copolymer; and the polycarbonate-segmented polyurethane elastomer. It is more favorable that each of these polymeric materials has a loss tangent not less than 0.4 at 25°C.

The styrene-isoprene-styrene tri-block copolymer and its hydrogenated tri-block copolymer are commercially available under the trade name of Hibrar (produced by Kuraray Corp.). The ethylene-styrene copolymer is commercially available under the trade name of Index (produced by Dow Chemical Corp.). The polycarbonate-segmented polyurethane elastomer is commercially available under the trade name of Miractran E985PNA (produced by Nippon Miractran Corp.).

It is preferable that to-be-mixed polymeric materials are compatible with each other to allow the medical tube formed of a compound of two or more kinds of polymeric materials to have a demanded kinking resistance, strength, transparency and flexibility.

The use of compatible polymeric materials prevents macro-dispersion of domains in resin. As the stress does not concentrate on an interface between the domains, the tube can have a high kinking resistance and a high tensile strength. Further, because light is hardly scattered on the macro-interface between the domains, the tube is highly transparent. The polymeric materials of the same kind are compatible with each other. Some polymeric materials of different kinds are compatible with each other. All of the above-described polymeric materials of (1) - (5) have preferable compatibility.

The loss tangent of the medical tube is obtained by the following method of measuring its dynamic viscoelasticity in its longitudinal vibration similarly to the above-described method to be carried for the above-described catheter.

A method of measuring the dynamic viscoelasticity of the polymeric material by means of torsional vibration is known. But this method is incapable of evaluating the kinking resistance of the polymeric material. It is preferable to evaluate the kinking resistance of the polymeric material by stretching and compressing the medical tube longitudinally, namely, by measuring its dynamic viscoelasticity in its longitudinal vibration. When an external force is applied to the medical tube, the medical tube yields thereto, i.e., the medical tube kinks. When the medical tube kinks, its outer side elongates, whereas its inner side is compressed.

The dynamic viscoelasticity of the polymeric material in its longitudinal vibration was measured on a dynamic viscoelastic analyzer DVA-225 manufactured by IKT Co. Ltd. by temperature rise method in the air at a measuring frequency of 10 Hz at a temperature rise rate of 5°C/min. A specimen was formed by cutting the catheter to a strip having 2mm in its widthwise direction and 25mm in its longitudinal direction.

Based on measured dynamic storage elastic modulus (E') and dynamic loss elastic modulus (E"), a loss tangent(tan$\delta$) was estimated by the following equation:

$$E^* = E' + iE''$$

$$\tan\delta = E''/E'$$

where E* is a complex modulus.

The kinking resistance and the maximum push-fit strength were measured by the following method:

The kinking resistance and the maximum push-fit strength were measured by using a compression tester 10 shown in Figs. 1 and 2. The compression tester 10 has a clamp 2a so installed at an upper portion thereof as to be movable vertically at a constant speed and a damp 2b fixed to a lower portion thereof. A medical tube having a predetermined length was set between the damps 2a and 2b. The compression tester 10 was so constructed that by compressing the medical tube axially, the change of a load applied to the medical tube was recorded on a chart. The kinking resistance and push-fit strength of the medical tube having a length 3 of 70mm were measured at a speed of 100mm/min. at a room temperature (25°C, dry) as follows. The medical tube used in the test had a size 12F having an inner diameter of φ2.6mm and an outer diameter of φ4.0mm.

As the medical tube was compressed axially, as shown in Figs. 1 and 2, a load applied to the medical tube changes. The change of the load is shown on the chart of Fig. 3. Upon axial compression of the medical tube, the load applied to the medical tube increases instantaneously. When the medical tube starts to leap, the load decreases (flexure start point 6). The load measured when the medical tube has started to bend is the maximum push-fit strength. As the compression continues, the lumen of the medical tube is crushed and starts to be closed (kinked). Thereby there is a great change in the reduction degree of the load to record an inflection point (kink start point 7) on the chart. Simultaneously with the closing of the lumen of the medical tube, the load is applied thereto constantly. At this time, an inflection point (kink point 8) is recorded on the chart. In the compression test, an operator measures the movement distance (mm) 4 of the clamp 2a from a position corresponding to a start point (start point 5) to a position corresponding to the closing (kink point 8) time of the lumen of the medical tube to determine the kinking resistance of the medical tube. In this manner, the kinking resistance and the maximum push-fit strength are measured.

EXAMPLES

Catheters of the examples of the present invention and the comparative examples were prepared to measure the loss tangents, kink resistances and maximum push-fit strengths thereof by using the above-described method.

Example 1

A polyurethane having a hard segment consisting of MDI and 1,4-butanediol and a soft segment consisting of polycarbonate glycol was prepared in a predetermined amount in such a way that [NCO]/[OH] = 1 to have a Shore hardness of 90A. This polyurethane was melt-extruded to form a catheter of example 1 (inner diameter: φ2.6mm, outer diameter: φ4.0mm, wall thickness: 0.7mm; size: 12F).

The loss tangent of the catheter of the example 1 at 25° was 0.34 and its kinking resistance was 28.5mm, which were preferable. The flexibility degree of the catheter was very dose to that of the same-size catheter made of polyvinyl chloride and had a touch like the catheter made of polyvinyl chloride. The maximum push-fit strength of the catheter was 3.9N, which means that the catheter allows an operator to have an easy insertion operation.

Example 2

A polyurethane having a hard segment consisting of HMDI and 1,4-butanediol and a soft segment consisting of polytetramethyl glycol was prepared in a predetermined amount in such a way that [NCO]/[OH] = 1 to have a Shore hardness of 90A. This polyurethane was melt-extruded to form a catheter of example 2 (inner diameter: φ2.6mm, outer diameter: φ4.0mm, wall thickness: 0.7mm; size: 12F).

The loss tangent of the catheter of the example 2 at 25° was 0.34 and its kinking resistance was 29.8mm, which were preferable. The flexibility degree of the catheter was very close to that of the same-size catheter made of polyvinyl chloride and had a touch like the catheter made of polyvinyl chloride. The maximum push-fit strength of the catheter was 4.6N, which means that the catheter allows the operator to have an easy insertion operation.

Example 3

A polyurethane having a hard segment consisting of MDI and 1,4-butanediol and a soft segment consisting of a copolymer of polytetramethyl glycol, polypropylene glycol and polyethylene glycol was prepared in a predetermined amount in such a way that [NCO]/[OH] = 1 to have a Shore hardness of 85A. This polyurethane was melt-extruded to form a catheter of example 3 (inner diameter: φ2.6mm, outer diameter: φ4.0mm, wall thickness: 0.7mm; size: 12F).

The loss tangent of the catheter of the example 3 at 25° was 0.15 and its kinking resistance was 38.3mm, which were preferable. The flexibility of the catheter was very close to that of the same-size catheter made of polyvinyl chloride and had a touch like the catheter made of polyvinyl chloride. The maximum push-fit strength of the catheter was 1.3N, which means that catheter allows the operator to have an easy insertion operation.

Example 4

A polyurethane having a hard segment consisting of MDI and 1,4-butanediol and a soft segment consisting of polycarbonate glycol was prepared in a predetermined amount in such a way that [NCO]/[OH] = 1 to have a Shore hardness of 80A. This polyurethane was melt-extruded to form a catheter of example 4 (inner diameter: φ2.6mm, outer diameter: φ4.0mm, wall thickness: 0.7mm; size: 12F).

The loss tangent of the catheter of the example 4 at 25° was 0.31 and its kinking resistance was 27.5mm, which were preferable. The flexibility degree of the catheter was very dose to that of the same-size catheter made of polyvinyl chloride and had a touch like the catheter made of polyvinyl chloride. The maximum push-fit strength of the catheter was 1.1N, which means that the catheter allows the operator to have an easy insertion operation.

Comparative Example 1

A catheter of comparative example 1 (inner diameter: $\phi$2.6mm, outer diameter: $\phi$4.0mm, wall thickness: 0.7mm; size: 12F) was formed of melt-extruded Septon 2007 ((SEPS), produced by Kuraray Corp.) having a Shore hardness of 80A.
The Shore hardness of the catheter of the comparative example 1 was 80A. Thus, the catheter had sufficient degree of flexibility as a urethral catheter. But the loss tangent of the catheter at 25° was 0.06, which was comparatively small. Thus it kinked readily. The front portion of the catheter having a low loss tangent leaped elastically in a high extent and did not have a touch like the catheter made of polyvinyl chloride. Because the front portion of the catheter is so elastic that there is a possibility that urine which has attached to its front portion is scattered in the periphery.

Comparative Example 2

A catheter of comparative example 2 (inner diameter: $\phi$2.0mm, outer diameter: $\phi$4.0mm, wall thickness: 1.0mm; size: 12F) was formed of silicon. Because the catheter was thick, it hardly kink when it was bent. But because the inner diameter of the catheter was small, a sufficient lumen could not be obtained therein. The loss tangent of the catheter at 25° was 0.12, which was not high. Thus, the catheter did not have a touch like the catheter made of polyvinyl chloride.

Comparative Example 3

A urethral catheter of comparative example 3 (inner diameter: $\phi$2.6mm, outer diameter: $\phi$4.0mm, wall thickness: 0.7mm; size: 12F) formed of latex was flexible and did not give physical disorder to the urethra. But the loss tangent of the catheter at 25° was 0.05, which was comparatively low. Thus it kinked readily. Further, because the catheter had a low loss tangent, it did not have a touch like the catheter made of polyvinyl chloride. Because the front portion of the catheter is so elastic that there is a possibility that urine which has attached to its front portion is scattered in the periphery.
The medical tubes of the present invention were utilized as catheters and medical tubes of the comparative examples were utilized as catheters to measure the loss tangents, kink resistances and maximum push-fit strengths thereof by using the above-described method. The maximum push-fit strength is an important item in utilizing the medical tube as the catheter.

Example 5

70 wt% of syndiotactic 1,2-polybutadiene and 30 wt% of a styrene-isoprene-styrene tri-block copolymer were mixed with each other to prepare a compound of polymeric materials (blendmer). The specification of the syndiotactic 1,2-polybutadiene is as follows: the amount of 1,2-butadiene repeating unit: 90%; produced by JSR Corp.; trade name: RB810; the loss tangent of a tube: 0.0953 and its storage elastic modulus (MPa): 9.35 when its dynamic viscoelasticity was measured at 25°C, 10Hz; the outer diameter of the tube: 4.0mm; the wall thickness of the tube: 0.7mm. The specification of the styrene-isoprene-styrene tri-block copolymer is as follows: the amount of styrene: 20%; the amount of 3,4-isoprene repeating unit: 70%; the loss tangent of a tube: 1.36 and its storage elastic modulus (MPa): 27.5 when its dynamic viscoelasticity was measured at 25°C, 10Hz; the outer diameter of the tube: 4.0mm; and the wall thickness of the tube: 0.7mm.
The compound was formed into a tube (catheter) of the example 5 having an inner diameter of 2.6mm, an outer diameter of 4.0mm and a wall thickness of 0.7mm.
The loss tangent of the tube (catheter), its storage elastic modulus and the kinking resistance at 25°C were 0.419, 20.9MPa and 40mm respectively. The flexibility of the catheter was very close to that of the same-size catheter made of polyvinyl chloride and the catheter had a touch like the catheter made of polyvinyl chloride. The maximum push-fit strength of the catheter was 2.4N. Thus, the catheter had high operability in inserting the catheter into the human body.

Example 6

80 wt% of an ethylene-octene 1 copolymer and 20 wt% of the styrene-isoprene-styrene tri-block copolymer were mixed with each other to prepare a compound of the catheter polymeric materials (blendmer). The specification of ethylene-octene 1 copolymer is as follows: trade name: Engage 8100; produced by Dupont Dow Elastomer Corp.; the loss tangent of a tube: 0.0538 and its storage elastic modulus (MPa): 12.0 when its dynamic viscoelasticity was measured at 25°C, 10Hz; the outer diameter of the tube: 4.0mm; and the wall thickness of the tube: 0.7mm. The specification of the styrene-isoprene-styrene tri-block copolymer is as follows: the amount of styrene: 20%; the amount of 3,4-isoprene repeating unit: 70%; the loss tangent of a tube: 1.36 and its storage elastic modulus (MPa): 27.5 when its dynamic viscoelasticity was measured at 25°C, 10Hz; the outer diameter of the tube: 4.0mm; and the wall thickness of the tube: 0.7mm.
The compound was formed into a tube (catheter) of the example 6 having an inner diameter of 2.6mm, an outer diameter of 4.0mm and a wall thickness of 0.7mm.

The loss tangent of the tube (catheter), its storage elastic modulus and the kinking resistance at 25°C were 0.163, 26MPa and 36mm respectively. The flexibility of the catheter was very close to that of the same-size catheter made of polyvinyl chloride and the catheter had a touch like the catheter made of polyvinyl chloride. The maximum push-fit strength of the catheter was 2.5N. Thus, the catheter had high operability in inserting the catheter into the human body.

Example 7

80 wt% of an ethylene-vinyl acetate copolymer and 20 wt% of the styrene-isoprene-styrene tri-block copolymer were mixed with each other to prepare a compound of polymeric materials (blendmer). The specification of ethylene-vinyl acetate copolymer is as follows: the amount of vinyl acetate: 28%; produced by Tosoh Corp.; trade name: Ultrasen 751; the loss tangent of a tube: 0.0644 and its storage elastic modulus (MPa): 21.5 when its dynamic viscoelasticity was measured at 25°C, 10Hz; the outer diameter of the tube: 4.0mm; and the wall thickness of the tube: 0.7mm. The specification of the styrene-isoprene-styrene tri-block copolymer is as follows: the amount of styrene: 20%; the amount of 3,4-isoprene repeating unit: 70%; the loss tangent of a tube: 1.36 and its storage elastic modulus (MPa): 27.5 when its dynamic viscoelasticity was measured at 25°C, 10Hz; the outer diameter of the tube: 4.0mm; and the wall thickness of the tube: 0.7mm.

The compound was formed into a tube (catheter) of the example 7 having an inner diameter of 2.6mm, an outer diameter of 4.0mm and a wall thickness of 0.7mm.

The loss tangent of the tube (catheter), its storage elastic modulus and the kinking resistance at 25°C were 0.178, 25.4 MPa and 36mm respectively. The flexibility of the catheter was very close to that of the same-size catheter made of polyvinyl chloride had a touch like the catheter made of polyvinyl chloride. The maximum push-fit strength of the catheter was 2.8N. Thus, the catheter had high operability in inserting the catheter into the human body.

Example 8

50 wt% of the ethylene-octene 1 copolymer and 50 wt% of an ethylene-styrene copolymer were mixed with each other to prepare a compound of polymeric materials (blendmer). The specification of ethylene-octene copolymer is as follows: trade name: Engage 8100; produced by Dupont Dow Elastomer Corp.; the loss tangent of a tube: 0.0538 and its storage elastic modulus (MPa): 9.35 when its dynamic viscoelasticity was measured at 25°C, 10Hz; the outer diameter of the tube: 4.0mm; and the wall thickness of the tube: 0.7mm. The specification of ethylene-styrene copolymer is as follows: produced by Dow Kagaku Corp.; trade name:

Index; the loss tangent of a tube: 0.545 and its storage elastic modulus (MPa): 38.2 when its dynamic viscoelasticity was measured at 25°C, 10Hz; the outer diameter of the tube: 4.0mm; and the wall thickness of the tube: 0.7mm.

The compound was formed into a tube (catheter) of the example 8 having an inner diameter of 2.6mm, an outer diameter of 4.0mm and a wall thickness of 0.7mm.

The loss tangent of the tube (catheter), its storage elastic modulus and the kinking resistance at 25°C were 0.335, 21.3 MPa and 40mm respectively. The flexibility of the catheter was very dose to that of the same-size catheter made of polyvinyl chloride and the catheter had a touch like the catheter made of polyvinyl chloride. The maximum push-fit strength of the catheter was 2.3N. Thus, the catheter had high operability in inserting the catheter into the human body.

Example 9

60 wt% of the ethylene-styrene copolymer, 5 wt% of the styrene-isoprene-styrene tri-block copolymer and 35 wt% of process oil were mixed with each other to prepare a compound of polymeric materials (blendmer). The specification of ethylene-styrene copolymer is as follows: produced by Dow Kagaku Corp.; trade name: Index; the loss tangent of a tube: 0.545 and its storage elastic modulus (MPa): 38.2 when its dynamic viscoelasticity was measured at 25°C, 10Hz; the outer diameter of the tube: 4.0mm; and the wall thickness of the tube: 0.7mm. The specification of the styrene-isoprene-styrene tri-block copolymer is as follows: the amount of styrene: 30%; the amount of 3,4-isoprene repeating unit: 10%; the loss tangent of a tube: 0.188 and its storage elastic modulus (MPa): 13.8 when its dynamic viscoelasticity was measured at 25°C, 10Hz; the outer diameter of the tube: 4.0mm; and the wall thickness of the tube: 0.7mm. The specification of the process oil is as follows: paraffin oil; produced by Idemitsu Kosan Corp.; and trade name: Diana Process Oil PW-380.

The compound was formed into a tube (catheter) of the example 9 having an inner diameter of 2.6mm, an outer diameter of 4.0mm and a wall thickness of 0.7mm.

The loss tangent of the tube (catheter), its storage elastic modulus and the kinking resistance at 25°C were 0.306, 14.4 MPa and 39mm respectively.

The flexibility of the catheter was very close to that of the same-size catheter made of polyvinyl chloride and the catheter had a touch like the catheter made of polyvinyl chloride. The maximum push-fit strength of the catheter was 1.5N. Thus, the catheter had high operability in inserting the catheter into the human body.

Example 10

50 wt% of a carbonate polyurethane elastomer and 50 wt% of an polyester-segmented-polyurethane elastomer were mixed with each other to prepare a compound of polymeric materials (blendmer). The specification of the carbonate polyurethane elastomer is as follows: produced by Nippon Miractoran Corp.; trade name: Miractoran E985PNAT; the loss tangent of a tube: 0.317 and its storage elastic modulus (MPa): 25.1 when its dynamic viscoelasticity was measured at 25°C, 10Hz; the outer diameter of the tube: 4.0mm; and the wall thickness of the tube: 0.7mm. The specification of the polyester-segmented-polyurethane elastomer is as follows: produced by Nippon Miractran Corp.; trade name: Miractran E185PNAT; the loss tangent of a tube: 0.126 and its storage elastic modulus (MPa): 13.3 when its dynamic viscoelasticity was measured at 25°C, 10Hz; the outer diameter of the tube: 4.0mm; and the wall thickness of the tube: 0.7mm.

The compound was formed into a tube (catheter) of the example 10 having an inner diameter of 2.6mm, an outer diameter of 4.0mm and a wall thickness of 0.7mm.

The loss tangent of the tube (catheter), its storage elastic modulus and the kinking resistance at 25°C were 0.223, 19.3 MPa and 38mm respectively. The flexibility of the catheter was very dose to that of the same-size catheter made of polyvinyl chloride and the catheter had a touch like the catheter made of polyvinyl chloride. The maximum push-fit strength of the catheter was 3.5N. Thus, the catheter had high operability in inserting the catheter into the human body.

Example 11

50 wt% of the syndiotactic 1,2-polybutadiene and 50 wt% of the styrene-isoprene-styrene tri-block copolymer were mixed with each other to prepare a compound of polymeric materials (blendmer). The specification of the syndiotactic 1,2-polybutadiene is as follows: the amount of 1,2-butadiene repeating unit: 90%; produced by JSR Corp.; trade name: RB810; the loss tangent of a tube: 0.0953 and its storage elastic modulus (MPa): 9.35 when its dynamic viscoelasticity was measured at 25°C, 10Hz; the inner diameter of the tube: 2.6mm; its outer diameter: 4.0mm; its wall thickness: 0.7mm. The specification of the styrene-isoprene-styrene tri-block copolymer is as follows: the amount of styrene: 20%; the amount of 3,4-isoprene repeating unit: 50%; the loss tangent of a tube: 0.385 and its storage elastic modulus (MPa):8.55 when its dynamic viscoelasticity was measured at 25°C, 10Hz; the outer diameter of a tube: 4.0mm; and the wall thickness of the tube: 0.7mm.

The compound was formed into a tube (catheter) of the example 11 having an inner diameter of 2.6mm, an outer diameter of 4.0mm and a wall thickness of 0.7mm.

The loss tangent of the tube (catheter), its storage elastic modulus and the kinking resistance at 25°C were 0.275, 10.2MPa and 42mm respectively. The flexibility of the catheter was very dose to that of the same-size catheter made of polyvinyl chloride and the catheter had a touch like the catheter made of polyvinyl chloride. The maximum push-fit strength of the catheter was 2.3N. Thus, the catheter had high operability in inserting the catheter into the human body.

Example 12

70 wt% of a propylene-ethylene random copolymer and 30 wt% of the isoprene block-hydrogenated styrene-isoprene-styrene tri-block copolymer were mixed with each other to prepare a compound of polymeric materials (blendmer). The specification of the propylene-ethylene random copolymer is as follows: produced by Nippon Polychem Corp.; trade name: Novatech FL25RC; the loss tangent of a tube: 0.0653 and its storage elastic modulus (MPa): 20.35 when its dynamic viscoelasticity was measured at 25°C, 10Hz; the outer diameter of a tube: 4.0mm; its wall thickness: 0.7mm. The specification of the isoprene block-hydrogenated styrene-isoprene-styrene tri-block copolymer is as follows: the amount of styrene: 20%; the amount of 3,4-isoprene repeating unit: 70%; the loss tangent of a tube: 1.4 and its storage elastic modulus (MPa): 30.5 when its dynamic viscoelasticity was measured at 25°C, 10Hz; the inner diameter of a tube: 2.6mm; its outer diameter: 4.0mm; and the wall thickness of the tube: 0.7mm.

The compound was formed into a tube (catheter) of the example 12 having an inner diameter of 2.6mm, an outer diameter of 4.0mm and a wall thickness of 0.7mm.

The loss tangent of the tube (catheter), its storage elastic modulus and the kinking resistance at 25°C were 0.475, 15.3 MPa and 39mm respectively. The flexibility of the catheter was very dose to that of the same-size catheter made of polyvinyl chloride and the catheter had a touch like the catheter made of polyvinyl chloride. The maximum push-fit strength of the catheter was 3.5N. Thus, the catheter had high operability in inserting the catheter into the human body.

Example 13

70 wt% of syndiotactic the 1,2-polybutadiene and 30 wt% of the isoprene block-hydrogenated styrene-isoprene-styrene tri-block copolymer were mixed with each other to prepare a compound of polymeric materials (blendmer). The specification of the syndiotactic 1,2-polybutadiene is as follows: the amount of 1,2-butadiene repeating unit: 90%; produced by JSR Corp.; trade name: RB810; the loss tangent of a tube: 0.0953 and its storage elastic modulus (MPa): 9.35 when its dynamic viscoelasticity was measured at 25°C, 10Hz; the outer diameter of a tube: 4.0mm; and its wall thickness: 0.7mm. The specification of the isoprene block-hydrogenated styrene-isoprene-styrene tri-block copolymer is as follows: the amount of styrene: 20%; the amount of 3,4-isoprene repeating unit: 70%; the loss tangent of a tube: 1.4 and its storage elastic modulus (MPa): 30.5 when its dynamic viscoelasticity was measured at 25°C, 10Hz; the inner diameter of a tube: 2.6mm; its outer diameter: 4.0mm; and the wall thickness of the tube: 0.7mm.

The compound was formed into a tube (catheter) of the example 14 having an inner diameter of 2.6mm, an outer diameter of 4.0mm and a wall thickness of 0.7mm.

The loss tangent of the tube (catheter), its storage elastic modulus and the kinking resistance at 25°C were 0.475, 10.2 MPa and 43mm respectively. The flexibility of the catheter was very close to that of the same-size catheter made of polyvinyl chloride and the catheter had a touch like the catheter made of polyvinyl chloride. The maximum push-fit strength of the catheter was 2.0N. Thus, the catheter had high operability in inserting the catheter into the human body.

Comparative Example 4

70 wt% of the syndiotactic 1,2-polybutadiene and 30 wt% of the isoprene block-hydrogenated styrene-isoprene-styrene tri-block copolymer were mixed with each other to prepare a compound of polymeric materials (blendmer). The specification of the syndiotactic 1,2-polybutadiene is as follows: the amount of 1,2-butadiene repeating unit: 90%; produced by JSR Corp.; trade name: RB810; the loss tangent of a tube: 0.0953 and its storage elastic modulus (MPa): 9.35 when its dynamic viscoelasticity was measured at 25°C, 10Hz; the outer diameter of a tube: 4.0mm; and its wall thickness: 0.7mm. The specification of the isoprene block-hydrogenated styrene-isoprene-styrene tri-block copolymer is as follows: the amount of styrene: 20%; the amount of 3,4-isoprene repeating unit: 50%; the loss tangent of a tube: 0.385 and its storage elastic modulus (MPa): 8.55 when its dynamic viscoelasticity was measured at 25°C, 10Hz; the outer diameter of a tube: 4.0mm; and the wall thickness of the tube: 0.7mm.

The compound was formed into a tube (catheter) of the comparative example 4 having an inner diameter of 2.6mm, an outer diameter of 4.0mm and a wall thickness of 0.7mm.

The loss tangent of the tube (catheter), its storage elastic modulus and the kinking resistance at 25°C were 0.132, 10 MPa and 25mm respectively. The maximum push-fit strength of the tube was 2.1N.

Unlike the same-size flexible catheter made of polyvinyl chloride, the tube (catheter) of the comparative example 4 leaped elastically in a high extent and was thus difficult to handle.

Comparative Example 5

70 wt% of the syndiotactic 1,2-polybutadiene and 30 wt% of the isoprene block-hydrogenated styrene-isoprene-styrene tri-block copolymer were mixed with each other to prepare a compound of polymeric materials (blendmer). The specification of the syndiotactic 1,2-polybutadiene is as follows: the amount of 1,2-butadiene repeating unit: 90%; produced by JSR Corp.; trade name: RB810; the loss tangent of a tube: 0.0953 and its storage elastic modulus (MPa): 9.35 when its dynamic viscoelasticity was measured at 25°C, 10Hz; the outer diameter of a tube: 4.0mm; and its wall thickness: 0.7mm. The specification of the isoprene block-hydrogenated styrene-isoprene-styrene tri-block copolymer is as follows: the amount of styrene: 20%; the amount of 3,4-isoprene repeating unit: 10%; the loss tangent of a tube: 0.188 and its storage elastic modulus (MPa): 13.8 when its dynamic viscoelasticity was measured at 25°C, 10Hz; the outer diameter of a tube: 4.0mm; and the wall thickness of the tube: 0.7mm.

The compound was formed into a tube (catheter) of the comparative example 5 having an inner diameter of 2.6mm, an outer diameter of 4.0mm and a wall thickness of 0.7mm.

The loss tangent of the tube (catheter), its storage elastic modulus and the kinking resistance at 25°C were 0.084, 15.3 MPa and 27mm respectively. The maximum push-fit strength of the tube was 2.1N.

Unlike the same-size flexible catheter made of polyvinyl chloride, the tube (catheter) of the comparative example 5 leaped elastically in a high extent and was thus difficult to handle.

Comparative Example 6

80 wt% of the ethylene-octene 1 copolymer and 20 wt% of the isoprene block-hydrogenated styrene-isoprene-styrene tri-block copolymer were mixed with each other to prepare a compound of polymeric materials (blendmer). The specification of the ethylene-octene 1 copolymer is as follows: trade name: Engage 8100; produced by Dupont Dow Elastomer Corp.; the loss tangent of a tube: 0.0538 and its storage elastic modulus (MPa): 12.0 when its dynamic viscoelasticity was measured at 25°C, 10Hz; the outer diameter of the tube: 4.0mm; and the wall thickness of the tube: 0.7mm. The specification of the isoprene block-hydrogenated styrene-isoprene-styrene tri-block copolymer is as follows: the amount of styrene: 30%; the amount of 3,4-isoprene repeating unit: 10%; the loss tangent of a tube: 0.188 and its storage elastic modulus (MPa): 13.8 when its dynamic viscoelasticity was measured at 25°C, 10Hz; the outer diameter of a tube: 4.0mm; and the wall thickness of the tube: 0.7mm.

The compound was formed into a tube (catheter) of the comparative example 6 having an inner diameter of 2.6mm, an outer diameter of 4.0mm and a wall thickness of 0.7mm.

The loss tangent of the tube (catheter), its storage elastic modulus and the kinking resistance at 25°C were 0.088, 13.8 MPa and 23mm respectively. The maximum push-fit strength of the tube was 2.4N.

Unlike the same-size flexible catheter made of polyvinyl chloride, the tube (catheter) of the comparative example 6 leaped elastically in a high extent and was thus difficult to handle. Because the polymeric materials were not compatible well with each other, the solution were untransparent and thus the content was unvisible.

The catheter of the present invention is made of a melt-extruded polymeric material consisting of non-polyvinyl chloride. The catheter has a loss tangent not less than 0.15, when the dynamic viscoelasticity thereof is measured at 25°C in a longitudinal vibration thereof. The catheter has a kinking resistance not less than 25mm, when the catheter having a length of 70mm is used under a dry condition at room temprature. Therefore, the catheter has a high kinking resistance in inserting it into a human body and holding it at a predetermined portion thereof.

A medical tube is made of a polymeric material consisting of non-polyvinyl chlorides. The medical tube consists of a compound of two or more kinds of polymeric materials. The medical tube has a loss tangent not less than 0.15, when the dynamic viscoelasticity thereof is measured at 25°C in a longitudinal vibration thereof. The medical tube has a kinking resistance not less than 25mm, when the medical tube having a length of 70mm is used under a dry condition at room temperature. The medical tube can be operated without giving a user a feeling of physical disorder, similarly to the conventional tube made of polyvinyl chloride. Further, the medical tube has a high kinking resistance.

**Claims**

1. A catheter which is made of a melt-extruded polymeric material consisting of non-polyvinyl chloride; has a loss tangent not less than 0.15, when a dynamic viscoelasticity thereof is measured at 25°C in a longitudinal vibration thereof; and has a kinking resistance not less than 25mm, when said catheter having a length of 70mm is used under a dry condition at room temperature.

2. A catheter according to claim 1, wherein a maximum push-fit strength of said catheter having a length of 70mm is in the range of 0.5N to 5N, when said catheter is used under a dry condition at room temperature.

3. A catheter according to claim 1 or 2, wherein said polymeric material has a Shore hardness in the range of 70 to 95A.

4. A catheter according to any one of claims 1 through 3, wherein said polymeric material is polyurethane.

5. A catheter according to claim 4, wherein said polyurethane comprises a diisocyanate component and a diol component as a hard segment and a polyglycol component as a soft segment.

6. A catheter according to claim 5, wherein said diisocyanate component consists of one or more diisocyanates selected from among 4,4'-diphenylmethane diisocyanate (MDI), 3,3'-diphenylmethane diisocyanate, toluene diisocyanate, 4,4'-dicyclohexylmethane diisocyanate (HMDI), hexamethylene diisocyanate and isophorone diisocyanate.

7. A catheter according to claim 5 or 6, wherein said diol component consists of one or more diols selected from among 1,4-butanediol, ethylene glycol, diethylene glycol, triethylene glycol, propylene glycol and 1,6-hexanediol.

8. A catheter according to any one of claims 5 through 7, wherein said polyglycol component consists of one or more glycols selected from among polyether glycol, polycarbonate glycol, polycaprolactone glycol and polyadipate glycol.

9. A catheter according to claim 8, wherein said polyether glycol consists of one or more glycols selected from among polytetramethylene glycol, polyethylene glycol and polypropylene glycol; a copolymer thereof; or a mixture thereof.

10. A catheter according to claim 4 or 5, wherein said polyurethane comprises any one of an elastomer selected from an elastomer comprising 4,4'-diphenylmethane diisocyanate (MDI) and 1,4-butanediol as a hard segment and polycarbonate glycol as a soft segment; an elastomer comprising 4,4'-dicyclohexylmethane diisocyanate (HMDI) and said 1,4-butanediol as a hard segment and polytetramethylene glycol as a soft segment; an elastomer comprising said 4,4'-diphenylmethane diisocyanate (MDI) and said 1,4-butanediol as a hard segment and polytetramethylene glycol as a soft segment or a mixture or a copolymer of said polytetramethylene glycol, polypropylene glycol and polyethylene glycol as a soft segment.

11. A catheter according to any one of claims 1 through 10, wherein said catheter is a urethral catheter.

12. A medical tube which is made of a compound of two or more kinds of polymeric materials each consisting of non-polyvinyl chloride; has a loss tangent not less than 0.15 when a dynamic viscoelasticity thereof is measured at 25°C in a longitudinal vibration thereof; and has a kinking resistance not less than 25mm when said medical tube having a length of 70mm is used under a dry condition at room temperature.

13. A medical tube according to claim 12, wherein one of two or more kinds of said polymeric materials has a loss tangent not less than 0.3 when a dynamic viscoelasticity of said medical tube is measured at 25°C in a longitudinal vibration thereof.

14. A medical tube according to claim 12 or 13, wherein one of two or more kinds of said polymeric materials consists of any one of 1,2-polybutadiene, an ethylene copolymer and a propylene copolymer.

15. A medical tube according to any one of claims 12 through 14, wherein one of two or more kinds of said polymeric materials consists of a block copolymer composed of a polymer block A consisting of an aromatic vinyl monomer as a main component thereof and a polymer block B consisting of a conjugated diene monomer as a main component thereof or a block copolymer of said polymer block A and said hydrogenated polymer block B.

16. A medical tube according to claim 12 or 13, wherein a compound of two or more kinds of said polymeric materials consists of any one of 1,2-polybutadiene, an ethylene copolymer and a propylene copolymer; and a block copolymer of a polymer block A consisting of an aromatic vinyl monomer as a main component thereof and a polymer block B consisting of a conjugated diene monomer as a main component thereof or a block copolymer of said polymer block A and said hydrogenated polymer block B.

17. A medical tube according to claim 12 or 13, wherein a compound of two or more kinds of said polymeric materials consists of an ethylene copolymer and a block copolymer of a polymer block A consisting of an aromatic vinyl monomer as a main component thereof and a polymer block B consisting of a conjugated diene monomer as a main component thereof or a block copolymer of said polymer block A and said hydrogenated polymer block B.

18. A medical tube according to any one of claims 14 through 17, wherein an amount of ethylene contained in said ethylene copolymer is in the range of 50 mol % to 95 mol %.

19. A medical tube according to any one of claims 14, 16, 17 and 18, wherein said ethylene copolymer is a copolymer of ethylene and an aromatic vinyl monomer.

20. A medical tube according to any one of claims 14, 16,17 and 18, wherein said ethylene copolymer is composed of any one of an ethylene-octene copolymer, an ethylene-styrene copolymer and an ethylene-vinyl acetate copolymer.

21. A medical tube according to claim 12 or 13, wherein a compound of two or more kinds of said polymeric materials is composed of 1,2-polybutadiene and a block copolymer of a polymer block A consisting of an aromatic vinyl monomer as a main component thereof and a polymer block B consisting of a conjugated diene monomer as a main component thereof or a block copolymer of said polymer block A and said hydrogenated polymer block B.

22. A medical tube according to any one of claims 15 through 21, wherein said block copolymer is a styrene-isoprene-styrene tri-block copolymer or an isoprene-hydrogenated tri-block copolymer formed by hydrogenating an isoprene block of said styrene-isoprene-styrene tri-block copolymer.

23. A medical tube according to claim 22, wherein said styrene-isoprene-styrene tri-block copolymer consisting of the polyisoprene block which has 3,4-isoprene repeating unit at not less than 45%.

24. A medical tube according to claim 12, wherein a compound of two or more kinds of said polymeric materials consists of an polyester-segmented polyurethane elastomer and a polycarbonate-segmented polyurethane elastomer.

25. A medical tube according to claim 12, wherein a compound of two or more kinds of said polymeric materials consists of an ethylene-octene copolymer and an ethylene-styrene copolymer.

26. A medical tube according to claim 25, wherein an amount of ethylene contained in said ethylene-styrene copolymer is in the range of 50 mol % to 95 mol %.

27. A medical tube according to any one of claims 12 through 26, wherein a compound of two or more kinds of said polymeric materials has a Shore hardness in the range of 60 - 95A.

28. A medical tube according to any one of claims 12 through 27, wherein said medical tube is a catheter.

29. A medical tube according to claim 28, wherein said catheter is a urethral catheter.

30. A medical tube according to any one of claims 12 through 29, wherein a maximum push-fit strength of said medical tube having a length of 70mm is in the range of 0.5 to 5N, when said medical tube is used under a dry condition at room temperature.

# FIG. 1

10

2a

1

3

2b

# FIG. 2

10

2a

4

1

2b

# FIG. 3

# FIG. 4

# FIG. 5

EP 1 149 598 A2

# FIG. 6

24